# EUROPEAN PATENT SPECIFICATION

(11) **EP 1 464 294 B1**
(45) Date of publication and mention of the grant of the patent: **11.07.2012**
(21) Application number: 04251617.9
(22) Date of filing: 22.03.2004
(51) Int. Cl.: A61B 17/74

(54) **Greater trochanter re-attachment device**
Vorrichtung zur Wiederbefestigung des Grossen Trochanters
Dispositif pour la réfixation du grand trochanter

(30) Priority: 02.04.2003 GB 0307648
(43) Date of publication of application: 06.10.2004
(73) Proprietor: Benoist Girard SAS, 14201 Hérouville-Saint-Clair Cédex (FR)
(72) Inventor: Delogé, Nicolas, 14440 Douvres la Délivrande (FR); Aux Epaules, Arnaud, 14750 Saint Aubin/Mer (FR); Asencio, Gérard, Rue des Bénédictins 30000 Nimes (FR)
(74) Representative: Bridge-Butler, Jeremy

(56) References cited:
- EP-A- 0 847 730
- EP-A- 0 955 013
- FR-A- 1 493 247
- FR-A- 2 792 823
- US-B1- 6 338 734

## Description

This invention relates to a greater trochanter re-attachment device in combination with a femoral prosthesis for use in transfemoral revision surgery. In the surgical technique which involves transfemoral osteotomy the femur is exposed along a proximal/distal line, the soft tissue (skin, muscle) being folded back on each side to expose the bone. The proximal end of the femur is now opened as a "window" and a femoral prosthesis is inserted into the bone canal.

When this surgery is performed in the transfemoral approach the lengthwise disection of the bone leaves the greater trochanter in place on a displaced portion of the bone. When the bone is closed again and the greater trochanter is in its correct position there is a loading on the closed bone where it has previously been opened and in known techniques the portions of the bone are wired together thus re-attaching the greater trochanter to the stem. This osteosynthesis occurs at the end of the operation. This is a critical aspect of the surgery because if the flap of bone containing the greater trochanter (the greater trochanter window) is not well attached the stresses on the stem will not be well distributed and a peak of stress can effect the distal tip of the stem leading to a cortex reaction, or a breakage of the stem, or of the screws if the stem is distally located.

In simpler first instance surgical procedures involving just the removal of the tip of the femur which carries the greater trochanter, it is known to re-attach that portion of the bone with rudimentary re-attachment devices. These devices can be simple because the re-attached tip of the femur is not subject to the kind of stresses applied to the length of the femur by a stem after transfemoral revision surgery like that described above.

In US 6338734, in the name of BURKE et al, a plate implant is used to re-attach the tip of the femur which carries the greater trochanter after it is severed during a first instance hip replacement procedure. The plate extends over the external surface of the greater trochanter, and is held in place with securing means in the form of wires, which pass over the plate and extend around most of the external surface of the femur. The wires are arranged normal to the plane at which the greater trochanter was severed, which plane is at approximately 30 degrees to the proximal/distal length of the femur. This is to ensure that the clamping force is oriented in the most efficient direction. In addition, in order to prevent axial movement of the plate the wires are passed through holes drilled in the femur on the opposite side of the femoral prosthesis implanted therein.

In FR 2792823, in the name of DE LA CAFFINIERE, a claw shaped clamp is placed over the top of the greater trochanter, which is held in place to secure the greater trochanter to the femur by a screw. The screw passes through an aperture drilled right through the greater trochanter and into a socket provided on the femoral prosthesis.

FR 1493247, in the name of NATIONAL RESEARCH DEVELOPMENT CORPORATION, and EP 0955013, in the name of PORTE, show further methods of re-attaching the removed tip of the femur carrying the greater trochanter in simple first instance surgical procedures. These cases relate to quite different surgical procedures in which nail components are applied to the exterior of the femur as opposed to stems inserted within it. As such, the re-attachment devices can be mounted to the exposed tails of the nail components. In NATIONAL RESEARCH DEVELOPMENT CORPORATION the greater trochanter portion of the femur is re-attached using a clamp overlying the greater trochanter and affixed to the shoulder of the nail component with a screw. In PORTE a flexible tie is passed around the greater trochanter portion of the bone, which is threaded through a guide hole in the shoulder of the nail component.

The present invention is intended to provide a trochanter re-attachment device in combination with a femoral prosthesis which will simplify the osteosynthesis and shorten the time required to perform it.

According to the present invention as defined by claim 1, there is provided a greater trochanter re-attachment device in combination with a femoral prosthesis for use in transfemoral revision surgery, in which the device comprises a bracket which when in use extends over the proximal external surface of the greater trochanter, and adjustable securing means on one free end of said bracket, which are adapted to extend around the external surface of the femur, and which when secured in position hold the greater trochanter in position in relation to the femoral prosthesis, characterised in that the other end of the bracket carries attachment means adapted for securing the device to the femoral prosthesis, and in that the bracket carries said adjustable securing means such that they extend around the external surface of the femur substantially normal to its proximal/distal length including the flap of bone containing the greater trochanter.

The attachment means may comprise a threaded screw adapted for insertion in a screw threaded socket in the shoulder of the femoral prosthesis.

The adjustable securing means are preferably in the form of one or more elongated flexible ties, for example a wire or ribbon, and two or more ties can be provided if required.

With the above described construction of bracket, it can have two arms which are substantially normal to each other, the first arm having a curved re-entrant shape to extend around and over the proximal end of the greater trochanter and having a bifurcated free end provided with said adjustable securing means, and the second arm carries the attachment means.

The bifurcated free ends of the first arm can each include a guide or guides to locate the adjustable securing means.

The bracket can be made from any convenient material, for example a synthetic plastics material or metal which is compatible with the requirements of the human body.

The threaded socket in the shoulder of the femoral prosthesis can be adapted to receive a stem impactor or extractor, or a targeting device with which it is used.

The invention can be performed in various ways but four embodiments and a description of a previously known method of attaching the greater trochanter during transfemoral revision surgery will now be described by way of example and with reference to the accompanying drawings in which :
Figure 1 is a pictorial view showing a known method of attaching the part of the bone of the femur which carries the greater trochanter in the opened (window) position and ready for re-attachment after transfemoral revision surgery;
Figure 2 is a side elevation of a greater trochanter re-attachment device according to the present invention;
Figure 3 is a plan view of the device shown in Figure 2;
Figure 4 is an end view of the device shown in Figures 2 and 3;
Figure 5 is a pictorial view of a femur with an installed femoral prosthesis after transfemoral revision surgery and showing the re-attachment device as shown in Figures 2, 3 and 4 in place;
Figure 6 is a pictorial plan view of the greater trochanter shown in Figure 5 with the re-attachment device in place;
Figure 7 is a top isometric view of an alternative construction; and
Figure 8 is a bottom isometric view of the alternative construction shown in Figure 7.

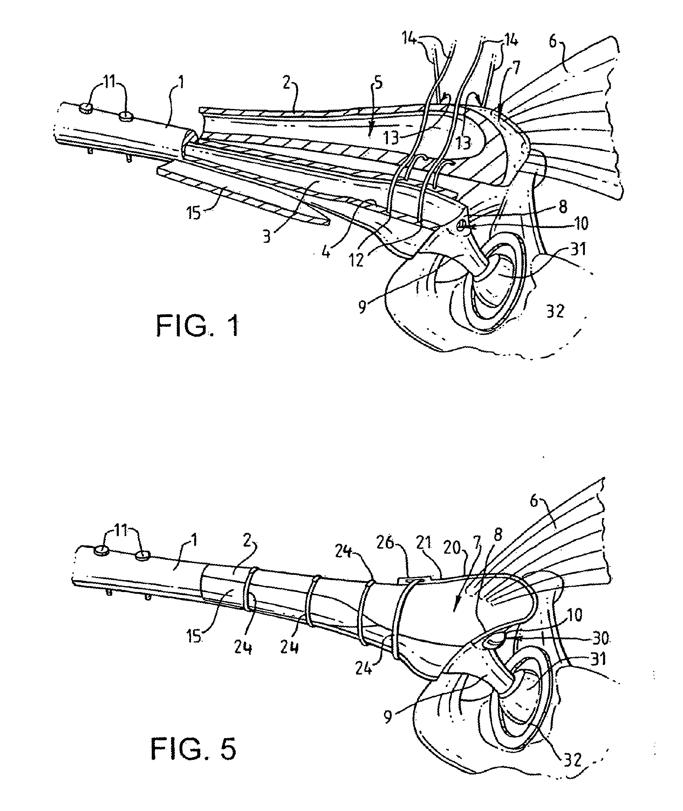

Figure 1 shows a known standard technique for closing the greater trochanter window after the installation of a femoral prosthesis. The stem 1 of the femur has been resected with three cuts along the axis of the bone and a transverse cut so that it can be opened, the portion of the bone containing the greater trochanter being indicated by reference numeral 2. The femoral prosthesis 3 has been inserted into one part 4 of the bone canal and another part of the canal which is in the portion 2 is indicated by reference numeral 5. The third central portion 15 of the bone is also provided which assists when the "window" is closed.

Reference numeral 6 indicates the muscles which are attached to the greater trochanter 7.

The femoral prosthesis 3 has a shoulder 8, a neck 9 and a screw threaded socket 10 is provided in the shoulder 8 to receive a stem impactor or extractor, or a targeting device. The distal end of the prosthesis is located in the bone canal 4 by means of screws 11.

During the surgery holes 12, 13 are drilled through the bone to accept binding wires 14 and when the "window" is closed these binding wires are pulled tight and clamped together.

Extra tie wires, not shown in Figure 1 but shown in Figure 5 are also usually employed to hold the bone together.

It will be appreciated that there are difficulties drilling the holes 12, 13, in threading the wires through them and then subsequently clamping them, and the technique is not without difficulties.

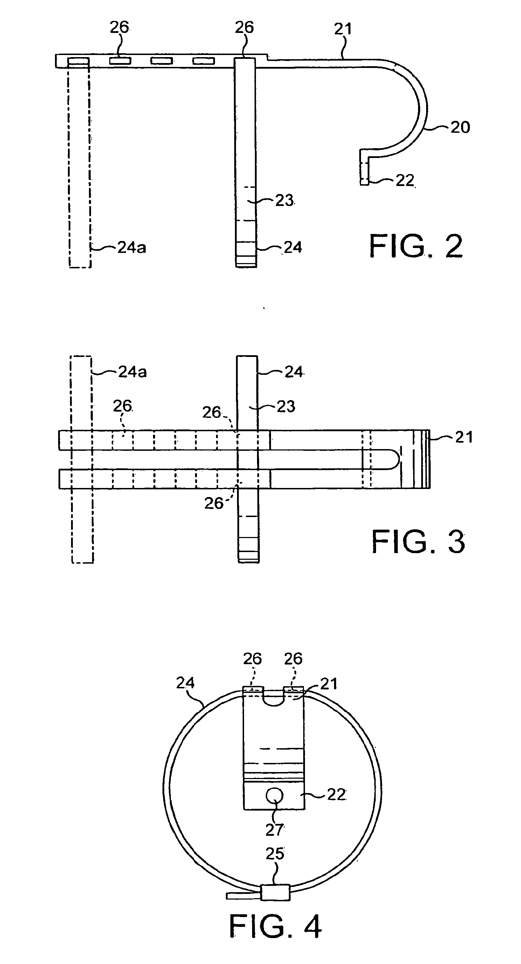

The greater trochanter re-attachment device for use in transfemoral revision surgery according to the present invention is shown in Figures 2, 3 and 4 and comprises a bracket 20 which has a first arm 21 which is substantially normal to a second arm 22. The first arm 21 has a curved re-entrant shape to extend around and over the proximal end of a greater trochanter. The free end of this arm 21 is bifurcated, as is most clearly shown in Figure 3 and Figure 5, and has adjustable securing means 23 which extend around the external surface of the bone substantially normal to its proximal distal length. The securing means is in the form of an elongated flexible tie 24 which can be, for example, a wire or ribbon. In the drawings it is shown in ribbon form with an end connector 25. The flexible tie extends through a guide in the bifurcated ends of the arm 21. In the arrangement shown in the drawings the guides are in the form of rectangular apertures 26 but they could be in the form of clips on the outer surface of the arm or any other convenient construction. As shown in Figures 2, 3 and 4 a series of apertures 26 are provided and the tie 24 can be used in any one of them. If appropriate one or more further ties, indicated by chain lines 26a, can be included.

If only one tie is used, or two close together, the unwanted bifurcated ends of the arm 21 can be removed.

The second arm 22 is provided with an opening 27 to receive attachment means for securing to the femoral prosthesis in the form of a threaded screw 30 (not shown in Figures 2, 3 or 4 but indicated in Figure 5). The screw 30 thus carries the adjustable securing means 24 via the bracket 20.

Figure 5 shows how the device shown in Figures 2, 3 and 4 is employed to re-attach the greater trochanter after transfemoral revision surgery.

The bracket 20 is first placed in position on the greater trochanter 7, the bifurcated ends of the arm 21 enabling the surgeon to pass them through the muscles 6 until the bracket is in the position shown in Figure 5. In this position the opening 27 in the second arm 22 is aligned with the screw threaded socket 10 in the prosthesis and a screw 30 is used as attachment means to secure the bracket to the shoulder 8 of the prosthesis 3.

The flexible tie 24 can now be passed through the apertures 26, assembled around the bone as shown in Figure 5 and tightened so that it acts as securing means extending around the external surface of the bone substantially normal to its proximal/distal length. If desired the flexible tie 24 could be pre-assembled on the end of the arm 21 prior to connecting the bracket to the shoulder 8 of the prosthesis 3.

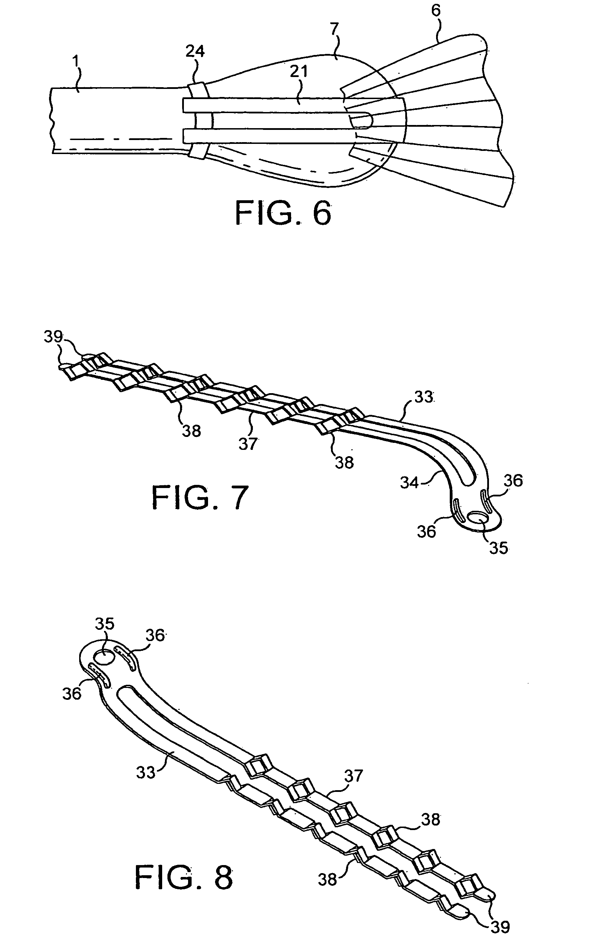

In the assembly shown in Figures 5 and 6 a single tie 24 is used in a single aperture 26 in the bifurcated arm 2 but if required a bracket having a number of apertures 26 could be employed with two or more ties 24a.

Additional external tie wires 24 can also be provided to hold the portion 2, part 4 and central portion 15 of the bone in place.

It will be appreciated that the present invention provides a simple device for rigidly holding the greater trochanter in place after the femoral prosthesis has been inserted.

Figures 1 and 5 show the head 31 of the prosthesis in place in an acetabular cup 32. The technique of being able to place the head 31 in position during transfemoral revision surgery is one of the advantages of this approach.

Figures 7 and 8 show an alternative construction for the bracket 20 and the same reference numerals are used to indicate similar parts to those shown in Figures 2 to 4. With this construction the bracket is indicated by reference numeral 33 and is made from a resilient material, for example a resilient metal strip. The bracket is initially formed with a single curve 34 which replaces the curved re-entrant shape shown in Figures 2 to 4. This end of the bracket has an opening 35 to receive the threaded screw 30 and the part of the bracket adjacent the opening 35 is again curved and pressed with troughs 36 to reinforce it.

The first arm 37 is again bifurcated and is split and dimpled on each side to provide a series of transversely extending openings 38 in each of the bifurcated arms 39.

The bracket is formed by a single pressing which provides the troughs 36 and the slits to allow the dimples to be produced.

It will be seen from the drawing that the arm 37 of the bracket shown in Figures 7 and 8 is somewhat longer than the arm 21 shown in Figures 2, 3 and 4. In use the bracket shown in Figures 7 and 8 is first attached to the prosthesis by the threaded screw 30. In this position the arm 37 will extend upwardly away from the bone at the greater trochanter 7. The surgeon can now however bend the bracket to provide a close and accurate fit due to the bracket's resilience. An elongated flexible tie 24, as shown in Figures 2, 3 and 4, can now be located through the appropriate openings 38 to hold the bracket in place on the greater trochanter 7. If desired a further elongated flexible tie 24a can be used as described with regard to Figures 2, 3 and 4.

## Claims

1. A greater trochanter re-attachment device (20) in combination with a femoral prosthesis (3) for use in transfemoral revision surgery, in which the device (20) comprises a bracket (20) which when in use extends over the proximal external surface of the greater trochanter (7), and adjustable securing means (23) on one free end of said bracket (20), which are adapted to extend around the external surface of the femur (1), and which when secured in position hold the greater trochanter (7) in position in relation to the femoral prosthesis (3), **characterised in that** the other end of the bracket (20) carries attachment means (30) adapted for securing the device (20) to the femoral prosthesis (3), and **in that** the bracket (20) carries said adjustable securing means (23) such that they extend around the external surface of the femur (1) substantially normal to its proximal/distal length including the flap of bone (2) containing the greater trochanter (7).

2. A combination (20, 3) as claimed in claim 1 in which the attachment means (30) comprises a threaded screw (30) adapted for insertion in a screw threaded socket (10) in the shoulder (8) of the femoral prosthesis (3).

3. A combination (20, 3) as claimed in claims 1 and 2 in which said adjustable securing means (23) are in the form of one or more elongated flexible ties (24).

4. A combination (20, 3) as claimed in claim 3 in which the one or more elongated flexible ties (24) are each in the form of a wire or ribbon (24).

5. A combination (20, 3) as claimed in claim 4 in which the bracket (20) has two arms (21, 22) which are substantially normal to each other, the first arm (21) having a curved re-entrant shape to extend around and over the proximal end of the greater trochanter (7) and having a bifurcated free end provided with said adjustable securing means (23).

6. A combination (20, 3) as claimed in claim 5 in which the bifurcated free ends of the first arm (21) each include a guide or guides (26) to locate the adjustable securing means (23).

7. A combination (20, 3) as claimed in any of the preceding claims 4, 5 or 6 in which the bracket (20) is made from a synthetic plastics material or metal which is compatible with the requirements of the human body.

8. A combination (20, 3) as claimed in claim 7 in which the bracket (20) is made from a resilient material which can be deformed to fit the bracket (20) to the shape of the greater trochanter (7) with which it is used.

9. A combination (20, 3) as claimed in claim 2 in which the threaded socket (10) is further adapted to receive a stem impactor or extractor or a targeting device with which it is used.

## Patentansprüche

1. Vorrichtung (20) zur Wiederbefestigung des großen Trochanters in Kombination mit einer Femoral-Prothese (3) zur Verwendung bei der Oberschenkel-Revisionschirurgie, wobei die Vorrichtung (20) eine Klammer (20), welche sich bei der Anwendung über die proximale Außenfläche des großen Trochanters (7) erstreckt sowie eine einstellbare Sicherungseinrichtung (23) an einem freien Ende dieser Klammer (20) aufweist, die derart ausgebildet ist, dass sie sich rund um die Außenfläche des Femurs (1) erstreckt und welche, wenn sie an Ort und Stelle angebracht ist, den großen Trochanter (7) in seiner Position in Bezug zur Femoralprothese (3) hält, **dadurch gekennzeichnet, dass** das andere Ende der Klammer (20) ein Befestigungselement (30) zur Befestigung der Vorrichtung (20) an der Femoralprothese (3) trägt sowie **dadurch**, dass die Klammer (20) die einstellbare Sicherungseinrichtung (23) derart hält, dass sich die letztere unter Einschluss des Knochensegmentes (2), welches den großen Trochanter (7) umfasst, rund um die Außenfläche des Femurs (1) und zwar im wesentlichen senkrecht zu dessen proximal-distaler Länge erstreckt.

2. Kombination (20, 3) nach Anspruch 1, bei welcher das Befestigungselement (30) eine Gewindeschraube (30) ist, die zum Einschrauben in eine Gewindebohrung (10) in der Schulter (8) der Femoralprothese (3) geeignet ist.

3. Kombination (20, 3) nach Anspruch 1 und 2, bei welcher die einstellbare Sicherungseinrichtung (23) die Form eines langgestreckten flexiblen Zugelementes oder mehrerer langgestreckter flexibler Zugelemente (24) hat.

4. Kombination (20, 3) nach Anspruch 3, bei welcher das bzw. die langgestreckte(n) flexible(n) Zugelement(e) (24) jeweils die Form eines Drahtes oder eines Bandes (24) hat (haben).

5. Kombination (20, 3) nach Anspruch 4, bei welcher die Klammer (20) zwei im Wesentlichen senkrecht zueinander angeordnete Arme (21, 22) aufweist, wobei der erste Arm (21) eine gebogene, einspringende Form hat, um sich rund um und über das proximale Ende des großen Trochanters (7) zu verstrecken sowie ein gegabeltes freies Ende, das mit der einstellbaren Sicherungseinrichtung (23) versehen ist.

6. Kombination (20, 3) nach Anspruch 5, bei welcher die gegabelten freien Enden des ersten Armes (21) jeweils eine Führung oder mehrere Führungen (26) zum Positionieren der einstellbaren Sicherungseinrichtungen (23) aufweisen.

7. Kombination (20, 3) nach einem der bisherigen Ansprüche 4, 5 oder 6, bei welcher die Klammer (20) aus einem synthetischen Kunststoffmaterial oder aus einem Metall hergestellt ist, wobei diese Materialien mit den Anforderungen des menschlichen Körpers verträglich sind.

8. Kombination (20, 3) nach Anspruch 7, bei welcher die Klammer (20) aus einem elastischen Material hergestellt ist, welches verformt werden kann, um die Klammer (20) an die Form des großen Trochanters (7), bei welchem sie angewandt wird, anzupassen.

9. Kombination (20, 3) nach Anspruch 2, bei welcher die Gewindebohrung (10) weiterhin zur Aufnahme einer Schaft-Einschlag- oder -Ausziehvorichtung oder einer dabei angewandten Zielvorrichtung geeignet ist.

## Revendications

1. Dispositif pour la refixation du grand trochanter (20) en combinaison avec une prothèse fémorale (3), destiné à être utilisé en chirurgie de révision fémorale, dans lequel le dispositif (20) comprend un support (20) qui, lorsqu'il est utilisé, s'étend sur la surface externe proximale du grand trochanter (7) et des moyens de fixation ajustables (23) sur une extrémité libre dudit support (20), qui sont adaptés pour s'étendre autour de la surface externe du fémur (1) et qui, lorsqu'ils sont fixés en position, maintiennent le grand trochanter (7) en position par rapport à la prothèse fémorale (3), **caractérisé en ce que** l'autre extrémité du support (20) supporte des moyens de fixation (30) adaptés pour fixer le dispositif (20) à la prothèse fémorale (3) et **en ce que** le support (20) supporte lesdits moyens de fixation ajustables (23) de sorte qu'ils s'étendent autour de la surface externe du fémur (1) sensiblement verticalement par rapport à sa longueur proximale/distale comprenant le lambeau d'os (2) contenant le grand trochanter (7).

2. Combinaison (20, 3) selon la revendication 1, dans laquelle les moyens de fixation (30) comprennent une vis filetée (30) adaptée pour l'insertion dans une douille filetée de vis (10) dans l'épaulement (8) de la prothèse fémorale (3).

3. Combinaison (20, 3) selon les revendications 1 et 2, dans laquelle lesdits moyens de fixation ajustables (23) se présentent sous la forme d'une ou de plusieurs attaches souples allongées (24).

4. Combinaison (20, 3) selon la revendication 3, dans laquelle les une ou plusieurs attaches souples allongées (24) se présentent chacune sous la forme d'un fil ou ruban (24).

5. Combinaison (20, 3) selon la revendication 4, dans laquelle le support (20) a deux bras (21, 22) qui sont sensiblement verticaux entre eux, le premier bras (21) ayant une forme rentrante incurvée pour s'étendre autour de et sur l'extrémité proximale du grand trochanter (7) et ayant une extrémité libre bifurquée avec lesdits moyens de fixation ajustables (23).

6. Combinaison (20, 3) selon la revendication 5, dans laquelle les extrémités libres bifurquées du premier bras (21) comprennent chacune un guide ou des guides (26) pour positionner les moyens de fixation ajustables (23).

7. Combinaison (20, 3) selon l'une quelconque des revendications 4, 5 ou 6 précédentes, 1 dans laquelle le support (20) est réalisé à partir d'une matière plastique synthétique ou d'un métal qui est compatible avec les besoins du corps humain.

8. Combinaison (20, 3) selon la revendication 7, dans laquelle le support (20) est réalisé à partir d'une matériau élastique qui peut être déformé pour adapter le support (20) à la forme du grand trochanter (7) avec lequel il est utilisé.

9. Combinaison (20, 3) selon la revendication 2, dans laquelle la douille filetée (10) est en outre adaptée pour recevoir un impacteur ou extracteur de tige ou un dispositif de ciblage avec lequel elle est utilisée.
